# EUROPEAN PATENT SPECIFICATION

(11) **EP 1 366 185 B1**
(45) Date of publication and mention of the grant of the patent: **27.12.2006**
(21) Application number: 01959799.6
(22) Date of filing: 10.07.2001
(51) Int. Cl.: C12Q 1/68

(54) **METHODS AND COMPOSITIONS FOR PERIOPERATIVE GENOMIC PROFILING**
VERFAHREN UND ZUSAMMENSETZUNGEN ZUR BESTIMMUNG VON PERIOPERATIVEN GENOMPROFILE
METHODES PERMETTANT D'ETABLIR UN PROFIL GENOMIQUE PERI-OPERATOIRE ET COMPOSITIONS AFFERENTES

(30) Priority: 11.07.2000 US 613887
(43) Date of publication of application: 03.12.2003
(73) Proprietor: Hogan, Kirk, Madison, WI 53705 (US)
(72) Inventor: Hogan, Kirk, Madison, WI 53705 (US)
(74) Representative: Keussen, Christof
(86) International application number: PCT/US2001/041331
(87) International publication number: WO 2002/006536

(56) References cited:
- WO-A-99/44062
- US-A- 5 413 907
- TANAKA T ET AL: "PROGNOSTIC DISCRIMINATION AMONG NEUROBLASTOMAS ACCORDING TO HA-RAS/TRK A GENE EXPRESSION A COMPARISON OF THE PROFILES OF NEUROBLASTOMAS DETECTED CLINICALLY AND THOSE DETECTED THROUGH MASS SCREENING" CANCER, AMERICAN CANCER SOCIETY, PHILADELPHIA, PA, US, vol. 83, no. 8, 15 October 1998 (1998-10-15), pages 1626-1633, XP001027462 ISSN: 0008-543X
- LOWE S ET AL: "Anesthetic implications of the child with homocystinuria." JOURNAL OF CLINICAL ANESTHESIA. UNITED STATES 1994 MAR-APR, vol. 6, no. 2, March 1994 (1994-03), pages 142-144, XP001120069 ISSN: 0952-8180
- SCHORGE JOHN O ET AL: "Clinically significant venous thromboembolism after gynecologic surgery." JOURNAL OF REPRODUCTIVE MEDICINE, vol. 44, no. 8, August 1999 (1999-08), pages 669-673, XP001120049 ISSN: 0024-7758
- BADNER NEAL H ET AL: "The use of intraoperative nitrous oxide leads to postoperative increases in plasma homocysteine." ANESTHESIA & ANALGESIA, vol. 87, no. 3, 1998, pages 711-713, XP009000817 ISSN: 0003-2999
- FRITZE J ET AL: "HORIZONTAL-TWO-DIMENSIONAL ELECTROPHORESIS OF PLASMA BUTYRYLCHOLINESTERASE" BIOLOGICAL CHEMISTRY HOPPE-SEYLER, vol. 368, no. 3, 1987, pages 295-298, XP009000749 ISSN: 0177-3593
- MONNIER NICOLE ET AL: "Malignant-hyperthermia susceptibility is associated with a mutation of the alpha-1-subunit of the human dihydropyridine-sensitive L-type voltage-dependent calcium-channel receptor in skeletal muscle." AMERICAN JOURNAL OF HUMAN GENETICS, vol. 60, no. 6, 1997, pages 1316-1325, XP009004568 ISSN: 0002-9297 cited in the application
- BRANDT ANNEBILL ET AL: "Screening of the ryanodine receptor gene in 105 malignant hyperthermia families: Novel mutations and concordance with the in vitro contracture test." HUMAN MOLECULAR GENETICS, vol. 8, no. 11, October 1999 (1999-10), pages 2055-2062, XP001135004 ISSN: 0964-6906 cited in the application
- TSAI M Y ET AL: "HIGH PREVALENCE OF A MUTATION IN THE CYSTATHIONINE BETA-SYNTHASE GENE" AMERICAN JOURNAL OF HUMAN GENETICS, UNIVERSITY OF CHICAGO PRESS, CHICAGO,, US, vol. 59, no. 6, 1 December 1996 (1996-12-01), pages 1262-1267, XP000672710 ISSN: 0002-9297 cited in the application
- HARMON DAWN L ET AL: "Methionine synthase D919G polymorphism is a significant but modest determinant of circulating homocysteine concentrations." GENETIC EPIDEMIOLOGY, vol. 17, no. 4, November 1999 (1999-11), pages 298-309, XP009004569 ISSN: 0741-0395 cited in the application
- FROSST P ET AL: "A CANDIDATE GENETIC RISK FACTOR FOR VASCULAR DISEASE: A COMMON MUTATION IN METHYLENETETRAHYDROFOLATE REDUCTASE" NATURE GENETICS, NEW YORK, NY, US, vol. 10, no. 1, 1 May 1995 (1995-05-01), pages 111-113, XP000198175 ISSN: 1061-4036 cited in the application
- SACHSE CHRISTOPH ET AL: "Cytochrome P450 2D6 variants in a Caucasian population: Allele frequencies and phenotypic consequences." AMERICAN JOURNAL OF HUMAN GENETICS, vol. 60, no. 2, 1997, pages 284-295, XP009004570 ISSN: 0002-9297 cited in the application
- LA DU B N ET AL: "PROPOSED NOMENCLATURE FOR HUMAN BUTYRYLCHOLINESTERASE GENETIC VARIANTS IDENTIFIED BY DNA SEQUENCING" CELLULAR AND MOLECULAR NEUROBIOLOGY, NEW YORK, NY, US, vol. 11, no. 1, February 1991 (1991-02), pages 79-89, XP009000117 cited in the application

## Description

### FIELD OF THE INVENTION

The present invention relates to methods for perioperative genomic screening of subjects, in particular to perioperative screening for markers indicative of responses to anesthesia and other perioperative or operative treatments and procedures. The present invention also provides compositions for use in screening methods.

### BACKGROUND OF THE INVENTION

Although surgery saves many lives, surgical complications result in many instances of mortality and morbidity. Complications related to surgery and anesthesia include infections, excessive blood loss, thrombosis, nausea and vomiting, and anesthesia reactions. These complications result in increased hospitalization, delayed recovery from surgery, and sometimes even death. Reactions to anesthesia present an example of such complications.

The use of local, regional, and general anesthesia is necessary to prevent pain and keep patients safe and stable during surgery. There are many options for techniques of anesthesia and specific anesthetic drugs. The choice of anesthetic regimen, agent, and dose depends on the type of surgery or procedure, other current medications, and any underlying diseases or pre-dispositions that a patient may have. Nonetheless, approximately one in 170 patients have complications related to anesthesia and one in 2500 surgical deaths can be attributed to anesthesia related complications (Dan Med Bull., 41:319 [1994]). Many complications are not the result of provider error, but rather of system errors, such as inadequacy of diagnosis with existing technologies. It is estimated that system errors account for up to 88% of the total errors in clinical practice (Liang and Cullen, Anesthesiology, 91: 609 [1999]).

One anesthesia-related complication is malignant hyperthermia (MH). MH is an autosomal dominant trait that causes a severe, uncontrollable fever when anesthesia is administered. One in 5000 to one in 15,000 children and 1 in 50,000 adults experience MH in response to trigger anesthetics. Lack of prompt treatment can result in cardiac dysrrhythmia, renal failure and death. MH is treated with the specific antidote dantrolene sodium, however, the best intervention is prevention. If a patient is identified as being at before anesthesia and alternative anesthetic drugs selected that carry no MH risk. At-risk patients are rarely identified by a family history of anesthesia reactions or by previous anesthesia reactions in the patient. No conclusive, simple, diagnostic screening method is available.

Subjects with defects in the enzymes that metabolize local anesthetics and related compounds can have poor reactions when given such drugs before, during, or following surgery. For example, muscle relaxants commonly given in conjunction with anesthesia, such as succinylcholine or mivacurium, can cause prolonged paralysis and apnea in a patient after the patient has awoken from anesthesia. The paralysis, caused by mutations in the butrylcholinesterase gene (BChE), is inherited as an autosomal recessive trait. The only available treatment is artificial ventilation and sedation until the paralysis subsides (30 minutes to 8 hours). In addition, BChE is responsible for the metabolism of ester local anesthetics. Thus, mutations in BChE can also lead to delayed metabolism and possible toxicity when ester local anesthetics are used. Biochemical assays that measure BChE are costly, time consuming, and lacking in accuracy. No conclusive, rapid screening assay for BChE mutations is available.

In addition, subjects with mutations in Cytochrome P450 enzymes, which metabolize a variety of drugs commonly given in conjunction with surgical procedures, can have adverse reactions due either to the inability to activate or metabolize certain drugs (e.g., morphine derivatives and anti-dysrrhthmics). Complications can be avoided by substituting other medications or adjusting dosage.

Reactions to drugs given during surgery are not the only surgical complications. Complications can also arise in the recovery period following surgery. One serious post-surgical complication is sepsis, a systemic reaction caused by infection, characterized by arterial hypotension, metabolic acidosis, decreased systemic vascular resistance, tachypnea and organ dysfunction. Sepsis is a major cause of morbidity and mortality in humans and other animals. It is estimated that 400,000-500,000 episodes of sepsis resulted in 100,000-175,000 human deaths in the U.S. alone in 1991. Despite the major advances of the past several decades in the treatment of serious infections, the incidence and mortality due to sepsis continues to rise (Wolff, New Eng. J. Med., 324:486-488 [1991]). Subjects carrying the TNF2 allele of the TNFα gene have an increased susceptibility to sepsis and death from sepsis after surgery (Mira, JAMA 282:561-568 [1999]). However, the only available assays measure cytokine production directly and are expensive, transient, and inconvenient. No conclusive, rapid screening assay for the presence of the TNF2 allele is available.

A proper choice of anesthetic, related drugs, and other treatment factors can reduce complications and morbidity and mortality associated with surgery. Convenient, rapid assays predictive of risks of surgical complications are needed.

### SUMMARY OF THE INVENTION

The present invention is defined in the claims and relates to methods for perioperative genomic screening of subjects, in particular to perioperative screening for markers indicative of responses to anesthesia and other perioperative or operative treatments and procedures. The present invention also provides compositions for use in screening methods.

The present invention provides a method comprising: providing a sample obtained from a perioperative subject (*e.g.*, a tissue sample or genetic information); providing an assay for detecting two or more genetic markers wherein said markers comprises a mutation in two or more genes selected from the group consisting of BChE, CYP2D6, MTHFR, MS, CBS, F 5 Leiden, Prothrombin, RYR1, CACNA1S, and CPT 2, and subjecting the sample to the assay to generate a genomic profile for use in selecting an operative course of action, wherein said course of action is administration of anesthesia during a surgical procedure. In some embodiments, the anesthesia is a general anesthesia. In other embodiments, the anesthesia is a regional anesthesia. In some embodiments, the surgical procedure is non-invasive surgery. In other embodiments, the surgical procedure is invasive surgery.

In some embodiments, a genomic profile of the present invention comprises information pertaining to a pharmacodynamic risk. In other embodiments, the genomic profile comprises information pertaining to a pharmacokinetic risk. In further embodiments, the genomic profile comprises a presymptomatic diagnosis. In still further embodiments, the genomic profile comprises information pertaining to differential diagnosis of recognized co-existing diseases.

The present invention is also useful in a method comprising: providing a sample from a subject; providing an assay for detecting two or more genetic markers; and subjecting the sample to the assay to generate a genomic profile for use in selecting a medical treatment course of action. The sample can be from the subject in a time frame selected from: prior to undergoing a medical procedure, during a medical procedure, and following a medical procedure. The medical treatment can be non-surgical or surgical.

The present invention is further useful in a method, comprising: providing a sample from a subject; providing an assay for detecting two or more genetic markers associated with a pharmacological response; testing the sample in the assay to generate a genomic profile; and subjecting the subject to a surgical procedure, wherein the conditions for the procedure are based on the genomic profile. In some methods, the pharmacological response is to an anesthetic. In some methods, the condition for the procedure is the choice of anesthetic. In some methods, the two or more genetic markers are a mutation in two or more genes selected from the group consisting of BChE, CYP2D6, MTHFR, MS, CBS, F 5 Leiden, Prothrombin, RYR1, CACNA1S, and CPT 2.

The prevent invention is also useful in a system comprising an assay for generating a genomic profile of a perioperative subject where the assay comprises two or more genetic markers indicative of a medical course of action. In some systems, the course of action is a surgical course of action; in other systems, the course of action is administration of anesthesia during a surgery.

In some systems, the genomic profile comprises information pertaining to a pharmacodynamic risk. In other systems, the genomic profile comprises information pertaining to a pharmacokinetic risk. In some systems, the genomic profile comprises a presymptomatic diagnosis. In other systems, the genomic profile comprises information pertaining to a recognized co-existing disease.

### DESCRIPTION OF THE FIGURES

Figure 1 shows an outline of the flow of information in some embodiments of the present invention.
Figure 2 two shows the flow of a genomic sample and data generated from the sample in some embodiments of the present invention.

### GENERAL DESCRIPTION OF THE INVENTION

The present invention is defined in the claims and relates to methods for perioperative genomic screening of subjects, in particular to perioperative screening for markers indicative of responses to anesthesia and other perioperative or operative treatments and procedures. The present invention also provides compositions for use in screening methods.

The present invention provides a novel diagnostic tool currently unavailable in the surgical field. There is no current technology available that provides the information of the perioperative genomic profiles of the present invention. In fact, the current state of the surgical field is to reduce or eliminate perioperative testing. Thus, the present invention provides solutions for problems that have no available alternatives. In the absence of any competing technology for quantifying subject's genetic contributors to perioperative risk, alleles (*e.g.,* known alleles) are tested for (*e.g.,* using known methods) according to explicit selection categories and criteria *en bloc* to establish a genomic profile.

Historically, a broad screening panel (*e.g*., blood and urinalysis, EKG, and chest x-ray) were routinely performed prior to surgery. However, the current procedure is simply to ask a patient if they have had any previous difficulties with anesthesia or surgery. Sometimes, but not always, a cursory physical exam is also performed. The use of laboratory tests for relatively healthy patients has generally been reduced or eliminated. Reasons for elimination include the cost of screening tests, inaccuracy and lack of specificity, uncertainty as to how to alter treatment course of action in response to results, and future harm to patients by an invasive work-up in response to an incidental finding. If fact, current anesthesiology texts emphasize that recent studies indicate a lack of benefit from routine laboratory testing as a method of assessing patients preoperatively. These texts stress that optimal cost-benefit strategies can only be obtained when testing is reduced to only that indicated by history-taking (*See e.g.,* RD Miller, (ed.), *Anesthesia,* fifth edition, Churchill Livingstone, [2000], pgs. 824-883).

The present invention unites the disparate fields of medicine (*e.g.,* anesthesia and surgery) with genetics. The perioperative genomic testing of the present invention is in direct contrast to the panels of tests currently available. The perioperative genomic profiles of the present invention solve many of the problems described above that have led the movement away from preoperative laboratory tests. The perioperative genomic profiles are cost and time effective. Markers for inclusion are selected for their accuracy, specificity, and predictive value. The perioperative profiles of the present invention allow for the individualization of treatment options for each subject undergoing a medical or surgical procedure.

The testing of all preoperative patients with a panel assay allows the testing of markers that are rare but of utility. For example, an assay that includes many alleles, even if they are rare, will find a positive result in a sufficient number of subjects to make the assay worthwhile. The perioperative genomic panels of the present invention also provide the advantage of detection of additive and synergistic effects of conditions predicted by more than one allele. The perioperative genomic panels further provide the advantage of being able to distinguish between homozygous and heterozygous mutations.

In some embodiments, the markers predict a subject's response to anesthesia or other medications, including but not limited to those given in conjunctions anesthesia (*e.g.* defects in metabolism leading to complications such as paralysis or drug toxicity). In some embodiments, the markers predict a subject's risk for anesthesia-related complications (*e.g.*, malignant hyperthermia). In some embodiments, the markers predict potential complications that may arise during a subject's recovery from surgery (*e.g.,* risk of thrombosis or sepsis).

Markers are also selected for which the course of action can be altered in a time and cost-effective way to eliminate or reduce unwanted surgical complications. For example, a practitioner may chose a particular anesthetic or analgesic in order to avoid a life-threatening response. A negative result for a given marker therefore carries the potential to provide as much therapeutic utility as a positive result. For example, if a subject is found to have a marker indicative of not responding to a given drug used in emergency resuscitation, valuable time is not spend administering the drug. Additionally, if a subject is found not to have an underlying condition, that condition can be eliminated from those considered in making a differential diagnosis, decreasing the time before a life saving intervention can be initiated.

In some embodiments, the information obtained from the perioperative genomic profile is used to establish the subject's prognosis or odds of survival. In some embodiments, the information is used to select the safest and most efficacious surgical procedure. In some embodiments, the information is used to determine the level of post-surgical monitoring (*e.g.*, whether to send the subject home the same day or hospitalize overnight or whether or not to place the subject in an intensive care unit). For example, a subject found to be at risk for post-surgical complications can be carefully monitored (*e.g.*, in the intensive care unit) so that life-saving intervention can be started as soon as possible.

The information provided by the perioperative genomic profiles of the present invention is of utility to the clinician even if the profile is not available at the initiation of surgery (*e.g.*, in the case of emergency surgery where there is a short time period between diagnosis and surgery). If the genomic profile is completed during a surgical procedure, the course of treatment can be altered, if necessary, at this point. In addition, information relating to post-surgical recovery is useful even following surgery.

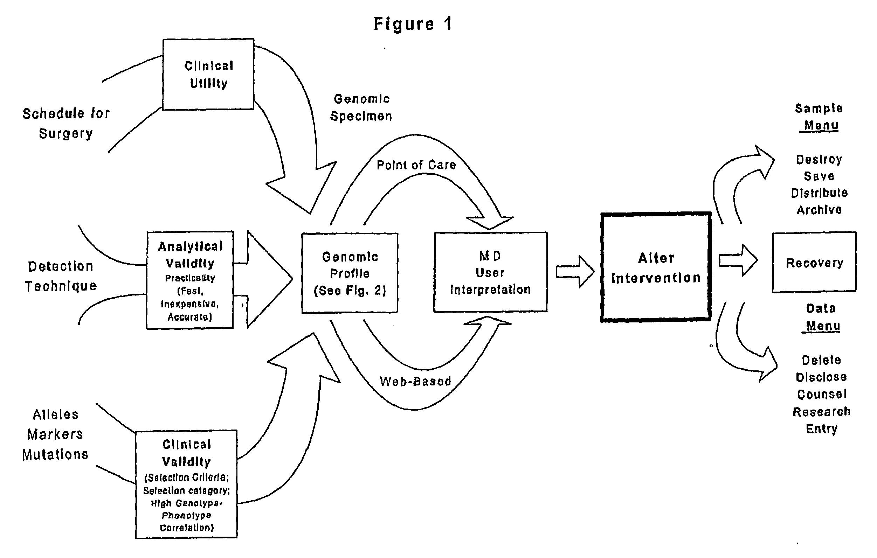

In some embodiments, the present invention further provides an integrated, electronic (*e.g.,* web-based) system for the gathering, processing, utilization, and distribution of genetic data relevant to a treatment course of action (See Figure 1 for an overview of the flow of information in some embodiments of the present invention). The present invention thus provides life and cost-saving information to practitioners on an accelerated scale relative to current diagnostics.

### DEFINITIONS

To facilitate an understanding of the invention, a number of terms are defined below.

The term "gene" refers to a nucleic acid (*e.g.,* DNA or RNA) sequence that comprises coding sequences necessary for the production of a polypeptide or precursor. The polypeptide can be encoded by a full length coding sequence or by any portion of the coding sequence so long as the desired activity or functional properties (*e.g.,* enzymatic activity, ligand binding, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The sequences that are located 5' of the coding region and that are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

In addition to containing introns, genomic forms of a gene may also include sequences located on both the 5' and 3' end of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, post-transcriptional cleavage and polyadenylation.

The term "wild-type" refers to a gene or gene product that has the characteristics of that gene or gene product when isolated from a naturally occurring source. A wild-type gene is that which is most frequently observed in a population and is thus arbitrarily designed the "normal" or "wild-type" form of the gene. In contrast, the terms "modified", "mutant", and "variant" refer to a gene or gene product that displays modifications in sequence and or functional properties (*i.e.,* altered characteristics) when compared to the wild-type gene or gene product. It is noted that naturally-occurring mutants can be isolated; these are identified by the fact that they have altered characteristics when compared to the wild-type gene or gene product.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

DNA molecules are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Therefore, an end of an oligonucleotide or polynucleotide, referred to as the "5' end" if its 5' phosphate is not linked to the 3' oxygen of a mononucleotide pentose ring, and as the "3' end" if its 3' oxygen is not linked to a 5' phosphate of a subsequent mononucleotide pentose ring. As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements that direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the terms "an oligonucleotide having a nucleotide sequence encoding a gene" and "polynucleotide having a nucleotide sequence encoding a gene," means a nucleic acid sequence comprising the coding region of a gene or, in other words, the nucleic acid sequence that encodes a gene product. The coding region may be present in either a cDNA, genomic DNA, or RNA form. When present in a DNA form, the oligonucleotide or polynucleotide may be single-stranded (*i.e.,* the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, *etc.* or a combination of both endogenous and exogenous control elements.

As used herein, the term "regulatory element" refers to a genetic element that controls some aspect of the expression of nucleic acid sequences. For example, a promoter is a regulatory element that facilitates the initiation of transcription of an operably linked coding region. Other regulatory elements include splicing signals, polyadenylation signals, termination signals, etc.

As used herein, the terms "complementary" or "complementarity" are used in reference to polynucleotides (*i.e.,* a sequence of nucleotides) related by the base-pairing rules. For example, for the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e.,* identity). A partially complementary sequence is one that at least partially inhibits a completely complementary sequence from hybridizing to a target nucleic acid and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.*, the hybridization) of a completely homologous sequence to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target that lacks even a partial degree of complementarity (*e.g.*, less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.,* the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (*See e.g.,* Anderson and Young, Quantitative Filter Hybridization, *in Nucleic Acid Hybridization* [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (*e.g.*, hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (*e.g.*, hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

"Amplification" is a special case of nucleic acid replication involving template specificity. It is to be contrasted with non-specific template replication (*i.e.,* replication that is template-dependent but not dependent on a specific template). Template specificity is here distinguished from fidelity of replication (*i.e.,* synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Template specificity is achieved in most amplification techniques by the choice of enzyme. Amplification enzymes are enzymes that, under conditions in which they are used, will process only specific sequences of nucleic acid in a heterogeneous mixture of nucleic acid. For example, in the case of Qβ replicase, MDV-1 RNA is the specific template for the replicase (Kacian *et al.,* Proc. Natl. Acad. Sci. USA, 69:3038 [1972]). Other nucleic acids will not be replicated by this amplification enzyme. Similarly, in the case of T7 RNA polymerase, this amplification enzyme has a stringent specificity for its own promoters (Chamberlin *et al.,* Nature, 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace, Genomics, 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (H.A. Erlich (ed.), *PCR Technology,* Stockton Press [1989]).

As used herein, the term "amplifiable nucleic acid" is used in reference to nucleic acids that may be amplified by any amplification method. It is contemplated that "amplifiable nucleic acid" will usually comprise "sample template."

As used herein, the term "sample template" refers to nucleic acid originating from a sample that is analyzed for the presence of "target" (defined below). In contrast, "background template" is used in reference to nucleic acid other than sample template that may or may not be present in a sample. Background template is most often undesired. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e*., a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. It is contemplated that any probe used in the present invention will be labelled with any "reporter molecule," so that is detectable in any detection system, including, but not limited to enzyme (*e.g.,* ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the method of K.B. Mullis U.S. Patent Nos. 4,683,195, 4,683,202, and 4,965,188, hereby incorporated by reference, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e*., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (*e.g.,* hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "amplification reagents" refers to those reagents (deoxyribonucleotide triphosphates, buffer, etc.), needed for amplification except for primers, nucleic acid template, and the amplification enzyme. Typically, amplification reagents along with other reaction components are placed and contained in a reaction vessel (test tube, microwell, etc.).

As used herein, the term "reverse-transcriptase" or "RT-PCR" refers to a type of PCR where the starting material is mRNA. The starting mRNA is enzymatically converted to complementary DNA or "cDNA" using a reverse transcriptase enzyme. The cDNA is then used as a "template" for a "PCR" reaction.

As used herein, the terms "restriction endonucleases" and "restriction enzymes" refer to bacterial enzymes, each of which cut double-stranded DNA at or near a specific nucleotide sequence.

As used herein, the term "antisense" is used in reference to RNA sequences that are complementary to a specific RNA sequence (*e.g.,* mRNA). Included within this definition are antisense RNA ("asRNA") molecules involved in gene regulation by bacteria. Antisense RNA may be produced by any method, including synthesis by splicing the gene(s) of interest in a reverse orientation to a viral promoter that permits the synthesis of a coding strand. Once introduced into an embryo, this transcribed strand combines with natural mRNA produced by the embryo to form duplexes. These duplexes then block either the further transcription of the mRNA or its translation. In this manner, mutant phenotypes may be generated. The term "antisense strand" is used in reference to a nucleic acid strand that is complementary to the "sense" strand. The designation (-) (*i.e*., "negative") is sometimes used in reference to the antisense strand, with the designation (+) sometimes used in reference to the sense (*i.e*., "positive") strand.

The term "isolated" when used in relation to a nucleic acid, as in "an isolated oligonucleotide" or "isolated polynucleotide" refers to a nucleic acid sequence that is identified and separated from at least one contaminant nucleic acid with which it is ordinarily associated in its natural source. Isolated nucleic acid is present in a form or setting that is different from that in which it is found in nature. In contrast, non-isolated nucleic acids are nucleic acids such as DNA and RNA found in the state they exist in nature. For example, a given DNA sequence (*e.g.,* a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid also includes, nucleic acid in cells ordinarily expressing a given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (*i.e.,* the oligonucleotide or polynucleotide may single-stranded), but may contain both the sense and anti-sense strands (*i.e.,* the oligonucleotide or polynucleotide may be double-stranded).

As used herein, a "portion of a chromosome" refers to a discrete section of the chromosome. Chromosomes are divided into sites or sections by cytogeneticists as follows: the short (relative to the centromere) arm of a chromosome is termed the "p" arm; the long arm is termed the "q" arm. Each arm is then divided into 2 regions termed region 1 and region 2 (region 1 is closest to the centromere). Each region is further divided into bands. The bands may be further divided into sub-bands. For example, the 11p15.5 portion of human chromosome 11 is the portion located on chromosome 11 (11) on the short arm (p) in the first region (1) in the 5th band (5) in sub-band 5 (.5). A portion of a chromosome may be "altered;" for instance the entire portion may be absent due to a deletion or may be rearranged (*e.g.,* inversions, translocations, expanded or contracted due to changes in repeat regions). In the case of a deletion, an attempt to hybridize (*i.e*., specifically bind) a probe homologous to a particular portion of a chromosome could result in a negative result (*i.e*., the probe could not bind to the sample containing genetic material suspected of containing the missing portion of the chromosome). Thus, hybridization of a probe homologous to a particular portion of a chromosome may be used to detect alterations in a portion of a chromosome.

The term "sequences associated with a chromosome" means preparations of chromosomes (*e.g.,* spreads of metaphase chromosomes), nucleic acid extracted from a sample containing chromosomal DNA (*e.g.,* preparations of genomic DNA); the RNA that is produced by transcription of genes located on a chromosome (*e.g.,* hnRNA and mRNA), and cDNA copies of the RNA transcribed from the DNA located on a chromosome. Sequences associated with a chromosome may be detected by numerous techniques including probing of Southern and Northern blots and *in situ* hybridization to RNA, DNA, or metaphase chromosomes with probes containing sequences homologous to the nucleic acids in the above listed preparations.

As used herein the term "coding region" when used in reference to a structural gene refers to the nucleotide sequences that encode the amino acids found in the nascent polypeptide as a result of translation of a mRNA molecule. The coding region is bounded, in eukaryotes, on the 5' side by the nucleotide triplet "ATG" that encodes the initiator methionine and on the 3' side by one of the three triplets which specify stop codons (*i.e.,* TAA, TAG, TGA).

As used herein, the term "purified" or "to purify" refers to the removal of contaminants from a sample. For example, nucleic acids contained in a sample (*e.g.,* blood or serum) are purified by removal of contaminating proteins and small molecules contained in the sample. Nucleic acids may be purified by any suitable method.

The term "recombinant DNA molecule" as used herein refers to a DNA molecule that is comprised of segments of DNA joined together by means of molecular biological techniques.

As used herein the term "portion" when in reference to a nucleotide sequence (as in "a portion of a given nucleotide sequence") refers to fragments of that sequence. The fragments may range in size from four nucleotides to the entire nucleotide sequence minus one nucleotide.

The term "recombinant protein" or "recombinant polypeptide" as used herein refers to a protein molecule that is expressed from a recombinant DNA molecule.

The term "native protein" as used herein to indicate that a protein does not contain amino acid residues encoded by vector sequences; that is the native protein contains only those amino acids found in the protein as it occurs in nature. A native protein may be produced by recombinant means or may be isolated from a naturally occurring source.

As used herein the term "portion" when in reference to a protein (as in "a portion of a given protein") refers to fragments of that protein. The fragments may range in size from four amino acid residues to the entire amino acid sequence minus one amino acid.

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (Sambrook *et al.,* Molecular Cloning: A Laboratory Manual, Cold Spring Harbor Press, NY, pp 9.31-9.58 [1989]).

The term "Northern blot," as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (Sambrook, *et al., supra,* pp 7.39-7.52 [1989]).

The term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabelled antibodies.

The term "antigenic determinant" as used herein refers to that portion of an antigen that makes contact with a particular antibody (*i.e*., an epitope). When a protein or fragment of a protein is used to immunize a host animal, numerous regions of the protein may induce the production of antibodies that bind specifically to a given region or three-dimensional structure on the protein; these regions or structures are referred to as antigenic determinants. An antigenic determinant may compete with the intact antigen (*i.e.,* the "immunogen" used to elicit the immune response) for binding to an antibody.

The term "transgene" as used herein refers to a foreign gene that is placed into an organism by introducing the foreign gene into newly fertilized eggs or early embryos. The term "foreign gene" refers to any nucleic acid (*e.g.,* gene sequence) that is introduced into the genome of an animal by experimental manipulations and may include gene sequences found in that animal so long as the introduced gene does not reside in the same location as does the naturally-occurring gene.

As used herein, the term "vector" is used in reference to nucleic acid molecules that transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The terms "overexpression" and "overexpressing" and grammatical equivalents, refers to the transcription and translation of a gene. Such transcription and translation may be *in vivo* or *in vitro.*

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "stable transfection" or "stably transfected" refers to the introduction and integration of foreign DNA into the genome of the transfected cell. The term "stable transfectant" refers to a cell that has stably integrated foreign DNA into the genomic DNA.

The term "transient transfection" or "transiently transfected" refers to the introduction of foreign DNA into a cell where the foreign DNA fails to integrate into the genome of the transfected cell. The foreign DNA persists in the nucleus of the transfected cell for several days. During this time the foreign DNA is subject to the regulatory controls that govern the expression of endogenous genes in the chromosomes. The term "transient transfectant" refers to cells that have taken up foreign DNA but have failed to integrate this DNA.

The term "calcium phosphate co-precipitation" refers to a technique for the introduction of nucleic acids into a cell. The uptake of nucleic acids by cells is enhanced when the nucleic acid is presented as a calcium phosphate-nucleic acid co-precipitate. The original technique of Graham and Van Der Eb (Graham and Van Der Eb, Virol., 52:456 [1973]), has been modified by several groups to optimize conditions for particular types of cells. The art is well aware of these numerous modifications.

A "composition comprising a given polynucleotide sequence" as used herein refers broadly to any composition containing the given polynucleotide sequence. The composition may comprise an aqueous solution. Compositions comprising polynucleotide sequences encoding a polypeptide or fragments thereof may be employed as hybridization probes. In this case, the polynucleotide sequences are typically employed in an aqueous solution containing salts (*e.g.,* NaCl), detergents (*e.g*., SDS), and other components (*e.g.*, Denhardt's solution, dry milk, salmon sperm DNA, etc.).

As used herein, the term "perioperative" refers to the time period around a surgical operation. The term encompasses the period before, during, and after a "surgical operation". The "perioperative" period begins when surgery is first contemplated (*e.g.,* when the patient is scheduled for surgery) and ends when recovery from surgery is complete (*e.g.*, when the services of a treating clinician are no longer required).

As used herein, the term "surgery" and related terms "surgical," "surgical operation," or "surgical intervention" refer to any medical procedure involving an incision into a tissue.

As used herein, the term "pre-surgical" refers to the period immediately before surgery. The "pre-surgical" period is generally utilized for preparing the subject for surgery. During the "pre-surgical" period, relevant testing and screening may be performed. It is not intended that the "pre-surgical" period is limited to a specific amount of time preceding surgery. In some cases, pre-surgical is any time period from several hours to several minutes before surgery (*e.g.,* in the case of urgent or emergency surgery). In other cases, the "pre-surgical" period may be several days or weeks prior to surgery (*e.g.,* in the case of non-emergency or elective surgery).

As used herein, the term "medical procedure" refers to any clinical or diagnostic procedure performed by a medical practitioner (*e.g.,* including, but not limited to a physician or physicians assistant, a nurse or nurse practitioner, or a veterinarian).

As used herein, the term "invasive surgery" refers to a "surgical procedure" requiring a large incision. Invasive surgery often requires a "general anesthetic." As used herein, the term "non-invasive surgery" refers to a "surgical procedure" that requires a minimal incision. "Non-invasive surgery" is often performed under "regional anesthesia" or "local anesthesia" supplemented with conscious sedation. "Non-invasive surgery" is often performed as an outpatient procedure.

As used herein, the term "anesthetic" refers to a medication that induces a reversible state of loss of sensation. "Anesthetics" sometimes cause a temporary state of loss of consciousness and paralysis. "Anesthetics" are often used during "surgery" to prevent pain.

As used herein, the term "local anesthesia" refers to an anesthesia that numbs a portion of the body (without affecting another portion of the body) for a short period of time. When "local anesthesia" is administered to a subject, the subject generally retains consciousness. Examples of "local anesthetics" include, but are not limited to bupivacaine and lidocaine.

As used herein, the term "regional anesthesia" refers to an anesthesia that numbs a portion of the body (without affecting another portion of the body) for up to several hours. When "regional anesthesia" is administered to a subject, the subject generally retains consciousness. Examples of "regional anesthesia" include, but are not limited to spinal or epidurally administered anesthesia.

As used herein, the term "general anesthesia" refers to an anesthesia that numbs the entire body for the duration of a "surgery." "General anesthesia" is generally administered continually (*e.g.,* intravenously or tracheally) throughout the procedure. When "general anesthesia" is administered to a subject, the subject generally does not retain consciousness. In addition, "general anesthesia" often requires artificial ventilation (*e.g.,* intubation).

As used herein, the term "genomic" relates to a "subject's" genetic makeup (*i.e*., their genome, or genes). For example, a "genomic profile" refers to a set of information about a given "subject's" genes (*e.g.,* the presence or absence of a specific set of mutations or "SNPs"). As used herein, the term "perioperative genomic profiling" refers to a "genomic profile" generated during the "perioperative" time period.

As used herein, the term "pharmacologic agent" refers to a compound (*e.g.,* an inorganic molecule or a protein) that has a physiological effect or "pharmacologic response" An example of a "pharmacologic agent" is a drug or a medication. As used herein, the term "pharmacodynamic risk" risk refers to a "subject's" risk of a clinical response of abnormal magnitude to a "pharmacologic agent." As used herein, the term "pharmacokinetic risk" refers to a "subject's" risk of abnormally absorbing, metabolizing (*e.g.,* not utilizing or utilizing too quickly), distributing, and excreting a "pharmacologic agent."

As used herein, the term "presymptomatic diagnosis" refers to the diagnosis of a medical condition or disease before the manifestation of symptoms. In some cases, the "presymptomatic diagnosis" diagnoses a genetic disease or predisposition.

As used herein, the term "differential diagnosis" as in "differential diagnosis of symptomatic disorders" refers to distinguishing between multiple disorders that may resemble one another outwardly (*e.g.,* have the same signs or symptoms), but have differing underlying causes and consequently require distinct interventions.

As used herein, the term "co-existing disease" refers to a condition towards which a "medical" or "surgical" procedure is not directed, but that may be relevant to certain aspects (*e.g.,* administration of anesthesia or analgesics) during a given "medical" or "surgical" procedure.

As used herein, the term "selecting a medical treatment course of action," or in the case of a "surgery," "selecting a surgical course of action" refers to care given during a "medical" or "surgical" procedure, including but not limited to choice of "pharmacologic agent," type of "anesthetic," or type of "surgery."

As used herein, the term "marker" refers to a reference point (*e.g.*, a point on a chromosome) for identification of a change or a mutation (*e.g.,* a nucleotide change). As used herein, the term "genetic marker" refers to a point (*e.g.*, on a chromosome, on a viral nucleic acid, or on a mitochondrial nucleic acid) for which a change (*e.g.*, a mutation or a polymorphism) causes a genotypic or phenotypic change. One example of a "genetic marker" is a "SNP."

As used herein, the terms "SNP," "SNPs" or "single nucleotide polymorphisms" refer to single base changes at a specific location in an organism's (*e.g.,* a human) genome. "SNPs" can be located in a portion of a genome that does not code for a gene. Alternatively, a "SNP" may be located in the coding region of a gene. In this case, the "SNP" may alter the structure and function of the protein in which it is located. In some instances, a "SNP" may affect an individuals response to a medical procedure or surgery (*e.g.*, response to an anesthetic or pain medication). The location and sequences of many "SNPs" are available in public databases (*See e.g.,* NCBI's dbSNP (http://www.ncbi.nlm.nih.gov/SNP/)) as well as private databases.

As used herein, the term "assay" refers to a method of detecting a "genetic marker." An assay may detect one or more "genetic markers" (*e.g.*, "SNPs"). Some assays may generate a "genomic profile."

As used herein, the term "sample" is used in its broadest sense. In one sense it can refer to a tissue or nucleic acid sample. In another sense, it is meant to include a specimen or culture obtained from any source. Biological samples may be obtained from animals (including humans) and encompass fluids, solids, tissues, and gases. Biological samples include, but are not limited to blood products, such as plasma, serum and the like. A "sample" can also be genetic information. For example, a subject's sequence data stored on a memory device (*e.g.*, a disk). These examples are not to be construed as limiting the sample types applicable to the present invention.

As used herein, the term "subject" refers an animal (*e.g.*, a human) undergoing a "medical" or "surgical" procedure. A "subject" may be a human or a non-human animal.

### DETAILED DESCRIPTION OF THE INVENTION

The present invention provides methods and compositions for perioperative genomic screening. In some embodiments, the genomic screening is designed to test for mutations and polymorphisms related to a subject's risk for anesthesia-related complications. In other embodiments, the perioperative genomic screen is designed to test for specific mutations or polymorphisms relevant to other types of surgery, surgical treatments, and surgical procedures including but not limited to cardiac surgery (*e.g.,* angiosplasty, bypass), brain surgery, abdominal surgery (*e.g.,* kidney or liver transplants), mastectomy, bone marrow transplants, bladder surgery, intestinal surgery (*e.g.*, colon or bowel surgery), lung surgery, spinal surgery, cosmetic and reconstructive surgery, gallbladder surgery, orthopedic surgery, and pediatric surgery (of all types). One skilled in the relevant art understands that the present invention encompasses perioperative genomic profiles for additional surgical techniques other than those listed above.

Markers for inclusion in perioperative genomic profiles are selected based on specific criteria. The sequence of the mutation or polymorphism, as well as the clinical outcome of carrying a mutant allele, should be known. In preferred embodiments, markers are selected for which there is no current alternative diagnostic test, or the available test is not suited for perioperative screening. In particularly preferred embodiments, markers are selected for which a clinical course of treatment can be altered in response to the presence or absence of a mutation or polymorphism.

Following selection of markers for inclusion in a given genomic profile, an assay for detection is provided. In some embodiments, the assay is a direct sequencing assay. In other embodiments, the assay is a fragment length polymorphism assay. In some preferred embodiments, the assay is a hybridization assay. In some preferred embodiments, the assay is a hybridization assay incorporating detection by enzymatic means. In other preferred embodiments, the assay is a MALDI-TOF mass spectrophotometric assay. However, the genomic profiles of the present invention find use with any detection method capable of detecting specific sequences and may be applied to detection methods developed in the future which may, or may not, rely on nucleic acid hybridization. In some embodiments, the process of selecting markers, performing detection assays, and distributing data to subjects and clinicians is organized by an integrated electronic (*e.g.,* web-based) system.

### I. Selection of Markers for Genomic Profile

In order to generate the perioperative genetic profiles of the present invention, markers are first selected for inclusion in the profile. The sequence of the markers should be known. In preferred embodiments, the markers are mutations in a given gene known to have an associated phenotype. Large amounts of sequence data and known mutations or polymorphisms are known and accessible. In preferred embodiments, markers are selected for their utility in providing information relevant to perioperative care.

### A. Sequence Data

In some embodiments of the present invention, the genetic markers are single nucleotide polymorphisms ("SNPs"). Known SNPs are available from public and private databases (see above). In other embodiments, the markers are mutations (*e.g.,* nucleotide deletions or insertions). In some embodiments, the markers represent splice variations. In other embodiments, the markers represent mutations in mitochondrial DNA.

In addition to known SNPs, a variety of nucleotide sequence information describing wild type and mutant alleles of a large number of genes is available in public databases including, but not limited to DbEST (http://www.ncbi.nlm.nih.gov/dbES); EBI/EMBL (http://www.ebi.ac.uk/mutations); EBI (http://www.ebi.ac.uk/ebi_home.html); EMBL (http://www.ebi.ac.uk/queries/queries.html); GDB (http://www.gdb.org/gdb/gdbtop.html); GeneCards (http://bioinformatics.weizmann.ac.il/cards/index.html); GeneClinics (http://www.geneclinics.org); Genethon (http://www.genethon.fr/genethon_en.html); GSDB (http://www.ncgr.org); HGP (http://www.ornl.gov/TechResources/Human_Genome/home.html); Human Gene Mutation Databoase (http://www.uwcm.ac.uk/uwcm/mg/search); NCBI (http://www.ncbi.nlm.nih.gov/); OMIM (http://www.ncbi.nlm.nih.gov/Omim/); PubMed (http://www.ncbi.nhn.nih.gov/PubMed/); Research Tools (NCBI) (http://www.ncbi.nhn.nih.gov/SCIENCE96/ResTools.html); RHdb (http://www.ebi.ac.uk/RHdb); Stanford Human Genome Center (http://www.shgc.stanford.edu/); HUGO (http://www.gene.ucl.ac.uklhugo); TIGR (http://www.tigr.org/); The National Human Genome Research Institute (http://www.nhgri.nih.gov/); The Whitehead Institute Center for Genome (http://www.genome.wi.mit.edu/); Unigene (http://www.ncbi.nlm.nih.gov/Unigene/index.html); University of Oklahoma (http://www.dnal.chem.ou.edu/index.html); and WEHI (http://wehih.wehi.edu.au/srs/srsc/). One skilled in the relevant art understands that nucleotide sequence data may be also be obtained from additional sources, including, but not limited to public and private databases; as well as experimentally.

### B. Criteria for Selection of Markers

In preferred embodiments of the present invention, the genetic markers selected for the perioperative genomic profile are tailored towards a specific medical or surgical procedure. The markers are selected based on several criteria, including but not limited to analytical validity, clinical validity, clinical utility, and commercial value.

In some embodiments of the present invention, markers are selected for their analytical validity (*e.g*., accuracy of detection using a particular detection technique). Markers are also selected based on their clinical validity, or their predictive effect (*e.g.,* the marker accurately predicts a subject's response to a specific aspect of the treatment). The sequence of all the mutations or polymorphisms to be tested should be available. For markers with multiple SNPs or mutations, it is preferred that the phenotypic outcome of each nucleotide change is known. It is also preferred that markers are selected for which the predisposition is unable to be determined (*e.g.,* cannot be determined cheaply or efficiently) through alternative means of detection, such as medical history, physical exam, or a non-genomic assay.

In some embodiments of the present invention, markers are selected for which the alternative treatment has little or no effect on the cost or inconvenience to the subject. Thus, markers are selected for which neither a false negative result (the original treatment is performed and the patient is in no worse a situation than if the assay had not been done) nor a false positive result (the alternative treatment is of equivalent cost and risk to original treatment) has a detrimental effect on subject outcome.

In some embodiments, the perioperative genomic profile includes two or more markers. In other embodiments, the perioperative genomic profile includes five or more markers. In some embodiments, the perioperative genomic profile includes 10 or more markers. In some preferred embodiments, the perioperative genomic profile includes 20 or more markers. In other preferred embodiments, the perioperative genomic profile includes 50 or more markers. In some particularly preferred embodiments, the perioperative genomic profile includes 100 or more markers. However, the utility of the assay is determined primarily by the predictive outcome of the individual markers or combination of markers, not the quantity of markers included.

In particularly preferred embodiments, markers are selected that provide information that can be used to alter the course of treatment (*i.e*., the markers have clinical utility). For example, if a subject is found to be predisposed to react poorly to one of several drugs commonly given during a surgical procedure, the practitioner may choose an alternative drug. Of particular utility are markers for predispositions for which an alternative treatment, equivalent in cost or ease of administration, can be substituted, thus saving lives and decreasing the number of expensive life-threatening traumas (*i.e.*, the inclusion of a given marker has the added advantage of having commercial value). In addition, markers are selected for which a negative result (*e.g.,* the absence of an underlying condition) has clinical utility (*e.g.,* aids in the differential diagnosis of a disease).

In some embodiments, the addition or subtraction of markers from the genomic profile is determined experimentally. For example, if it is determined that a marker does not correlate well with a subject's response to a given component of the treatment, the marker is subtracted. The inclusion of new markers may also be determined empirically. For example, if a new marker is found to have good predictive ability, alone or in combination with other markers, that marker is added to the genomic profile.

### C. Categories of Markers

In some preferred embodiments, markers that measure a subject's pharmacogenetic risk (response to pharmacological compound) are included. In some embodiments, markers for a subject's pharmacodynamic risk (a response of abnormal magnitude triggered by a pharmacological agent; *e.g*., malignant hyperthermia in response to anesthetic or bronchospasm unrelieved by an abnormal β1 adrenergic receptor response to a β1 agonist) are included in the perioperative genomic profile. In still further preferred embodiments, markers that predict a subject's pharmacokinetic response (abnormal adsorption, distribution, metabolism and excretion of a drug, resulting in overdose or lack of efficacy of a drug; *e.g.*, cytochrome P450 mutations that effect the metabolism of a variety of drugs) are included in the perioperative genomic profile.

In some preferred embodiments, markers with diagnostic utility are included in the perioperative genomic profile. In some preferred embodiments, markers that identify preexisting but non-symptomatic conditions that are relevant to the surgical procedure (*e.g.,* long QT syndrome or sickle cell trait that may manifest in response to surgery) are included in the perioperative genomic profile.

In additional preferred embodiments, markers are included that establish the differential diagnosis of symptomatic disorders that may resemble one another outwardly, but require different interventions during surgery. Examples include, but are not limited to classes of periodic paralyse or types of porphyria.

In some embodiments, the perioperative screening assay includes markers tailored to the specific surgical procedure being performed (*e.g.,* transplant recipients, cardiac surgery, or routine outpatient surgery). In some embodiments, the perioperative genomic profile includes markers unique to a subject in a certain group (*e.g*., age, ethnic background, gender).

In some embodiments, markers included in the genomic profile are haplotypes, or the natural variation within a gene unique to a given group of subjects (*e.g.,* a family of blood-relatives). Some haplotypes predict the response to a given pharmaceutical agent (*e.g.,* lack of response to a given drug).

In some embodiments, additional markers are included that are not specific for the surgical procedure being performed, but that predict general outcome of surgery and related procedures. Examples include, but are not limited to markers for aminoglycoside ototoxicity, APOε4, wound cytokines, sepsis risk (TNFα), blood groups, coagulation factors, and thrombosis risk. In some embodiments, the perioperative screening assay includes other tests unrelated to the genomic profile for the main surgical application, but relevant in the case of a complication requiring emergency intervention (*e.g.,* blood typing). In some embodiments, the perioperative genomic profile includes a unique genomic identifier (*e.g.,* a series of polymorphic non-coding SNPs), thus providing a secure, accurate internal reference for archiving and tracking genetic data specific to the particular subject.

### D. Applications and Interventions of Specific Markers

In some embodiments of the present invention, a genomic profile for perioperative screening of a subject's response to anesthesia (general, regional, or local) is generated. In preferred embodiments, markers are chosen that are predictive of not only a subject's response to a particular anesthesia, but also for known or unknown preexisting conditions that may influence a subject's response to a particular anesthesia or medication given in conjunction with anesthesia. In some preferred embodiments, the genomic profile additionally includes markers tailored towards the specific surgical procedure being performed.

In preferred embodiments involving perioperative screening for anesthesia responses, markers are selected for responses to specific anesthesia or drugs commonly given in conjunction with anesthesia (*e.g*., muscle relaxants or pain medications). In some embodiments, markers for mutations in the BChE gene are included in the perioperative genomic profile. Markers that are predictive of BChE deficiencies are known (*See e.g.,* La Du *et al.,* Cell. and Molec. Neurobiol., 11:79 [1991]). The only available assay for BChE is a biochemical assay that is too time-consuming and expensive to be included in routine perioperative screening. Furthermore, if a subject is found to contain a marker predictive of BChE deficiency, alternative drugs can easily be substituted without additional cost or inconvenience.

In some embodiments, markers for debrisoquine metabolism (*i.e*., Cytochrome P450) defects are included in the perioperative genomic profile. Defects in the CYP2D6 gene known to disrupt the pharmacokinetics of certain drugs have been described (*See e.g*., Sachse *et al.,* Am. J. Hum. Genet., 60:284 [1997]). Current biochemical assays for CYP2D6 mutations are too expensive and inconvenient to be included in perioperative screening. If a subject's predisposition to impaired or accelerated P450 metabolism is known, adverse drug reactions can easily be avoided by substituting other medications or adjusting dosages.

In addition, in some embodiments, markers for additional defects related to drug metabolism, including, but not limited to susceptibility to nitrous oxide toxicity or homocysteinemia associated with nitrous oxide (*e.g.,* mutations in cystathione β synthase, MTHFR, and methionine synthase genes) are also included in perioperative genomic profiles. In some embodiments, markers identifying subjects with underlying conditions that make them likely to respond poorly to anesthesia are also included in the perioperative genomic profile. For example, in some embodiments, markers for malignant hyperthermia (MH) are included in the genomic profile. Mutations predictive of MH are known in the art (*See e.g.,* Vladutiu *et al.,* Am J. Hum. Genet., 29:A5 [1998]; Monnier *et al.,* Am. J. Hum. Genet., 60:1316 [1997]). In addition, the only available diagnostic test for MH is an expensive *in vitro* contracture test requiring a muscle sample (*See e.g.,* Brandt *et al.,* Hum. Mol. Genet., 8:2055 [1999]). Furthermore, effective alternative treatments to prevent MH are available. If a subject is found to have a marker predictive of increased risk for MH, anesthetics known to trigger MH are avoided. In addition, the subject may be given dantrolene to prevent MH.

In some embodiments, markers for genetic diseases that may not be symptomatic, but may nonetheless effect the response to anesthesia, are also included. For example, markers for inherited arrthymogenic disorders (*See e.g.,* Priori *et al.,* Circulation, 99:518 [1999]) are included. One inherited arrthymogenic disorder is long QT syndrome, characterized by abnormally prolonged ventricular repolarization and a high risk of malignant ventricular tachyarrhythmias. Periods of high physical stress (*e.g.,* surgery and anesthesia) can trigger an attack in susceptible individuals. Identification of individuals with a marker predictive of long QT syndrome allows the practitioner to more closely monitor the individual for signs of cardiac abnormalities, avoid aggravating drugs and treat before refractory rhythms arise.

In some embodiments, perioperative genomic profiles include markers for blood coagulations proteins or platelet deficiencies (*e.g.,* methylene tetrahydrofolate reductase, methionine synthase, cystathione β synthase, factor V Leiden, and prothrombin) known to increase or to descrease the risk of thrombosis (blood clots). Many incidences of venous thrombosis are associated with surgery or other traumas and result in expensive therapies and morbidity. Approximately 50% of all thrombosis are hereditary (Brick, Seminars in Thrombosis and Hemostatis, 25:251 [1999]). Mutations and polymorphisms in these genes known to increase the risk of thrombosis have been identified (*See e.g.,* Frosst *et al.,* Nature Genet., 10:111 [1995]; Harmon *et al.,* Genet. Epidemiol., 17:298 [1999]; Tsai *et al.,* Am. J. Hum. Genet., 59:1262 [1996]; Simoni *et al.,* New Eng. J. Med., 336:399 [1997]; DeStefano *et al.,* New Eng. J. Med., 341:801 [1999]). If a subject is identified as being at risk for thrombosis, an alternative anesthesia or medication can be chosen. Prophylactic treatment (*e.g*., anti-coagulation medications, positioning, and compression devices) and closer monitoring can reduce the incidence and severity of thrombus.

In some embodiments, markers specific for coagulation defects (predictive of an increased risk of bleeding and associated stroke) are included in the perioperative genomic profile. Examples include, but are not limited to polymorphisms in tissue plasminogen activator (TPA), PAI-1, and fibrinogen. If a patient is found to be at increased risk for bleeding, specific post-surgical monitoring can be implemented to allow for early intervention. Additionally, pharmaceutical agents with a decreased risk of aggravating potential bleeding can be utilized.

In some embodiments, markers for polymorphisms in platelet surface adhesion molecules (*e.g.*, GP IIb/IIIa fibrinogen adhesion site), endothelial function, and inflammation (cytokines) are included in the perioperative genomic profile. Polymorphisms in these factors may be indicative of an increased risk for myocardial infarction (MI; heart attack). If a subject is found to have a marker indicative of an increased risk of an MI, appropriate pharmaceutical agents can be chosen for prevention or intervention and the patient can be specifically monitored for signs of a MI.

In some embodiments, markers are included for additional underlying conditions that may influence the choice of anesthesia or other management. Examples and altered courses of action include but are not limited to idiopathic hypertrophic subaortic stenosis (*e.g.,* avoid positive inotropes), dilated cardiomyopathy (*e.g.,* avoid negative inotropes), antitrypsin deficiency (*e.g.,* closely monitor for pulmonary complications), hemochromatosis (*e.g.,* avoid transfusions), Leber's optic atrophy (*e.g.*, avoid sodium nitroprusside), sickle trait, and thalassemia (*e.g*., closely monitor for anemia) are included in the perioperative genomic profile. In some embodiments, markers for co-existing diseases that may affect an individual's response to a certain anesthesia are included (*e.g.*, class of periodic paralysis [affects decision to avoid or administer potassium], or type of porphyria [affects decision to avoid or administer sodium thiopental]).

In some preferred embodiments, the perioperative genomic profile further includes markers specific for the selection of a given surgical procedure. For example, subjects undergoing cardiopulmonary bypass are tested for apolipoprotein E alleles. If a patient is found to have the E-ε4 allele (indicative of an increased risk of postoperative decline in cognitive function), a non-bypass procedure can be implemented (*e.g.*, minimally invasive, beating heart surgery, and off-pump bypass coronary artery grafting using mini-thoracotomy or mini-sternotomy approaches and a pressure-plate type stabilizer).

In addition, in some further embodiments, tests for markers involving further general surgical variables including, but not Limited to wound healing factors, cytokines, and antibiotic toxicity predisposition are also included. In some embodiments, markers for general genomic variables, including but not limited to blood serotype (*See e.g.,* Yamamoto *et al.,* Nature, 345:229 [1990] for specific markers) and predisposition to allergy (*e.g.,* to antibiotics or latex) are included. In some embodiments, markers that affect the course of emergency intervention are included (*e.g.,* lack of response to β-adrenergic bronchodilators or blood serotype) are included in the perioperative genomic profile. In some embodiments, markers are included for pathogenic infections that may effect response to surgery (*e.g.,* Hepatitis B virus and Hepatitis C virus).

In still further embodiments, markers predictive of possible complications during recovery from surgery, including, but not limited to, markers for a predisposition to sepsis (*e.g.*, TNF allele) are included. The TNF2 allele of TNFα is associated with an increased severity of sepsis. If a subject is found to have the TNF2 allele, intensive care monitoring post-surgery can be increased, decreasing the chance of death from sepsis. In addition, the practitioner may use the presence of the TNF2 allele as a factor in choosing a non-surgical treatment with a lower risk of sepsis. In some embodiments, markers for pathogens known to be responsible for causing septic infections (*e.g.,* bacterial DNA present in the bloodstream) are included in the perioperative genomic profile. One skilled in the relevant art understands that additional markers of utility to perioperative treatment can be included in the aforementioned perioperative genomic profiles.

### II. Assays for Generating Genomic Profiles

Once the particular SNPs and mutations have been determined for a given perioperative genomic panel, a profile is generated. Genomic profiles are generated through the detection of SNPs and mutations in a DNA sample (*e.g.,* a tissue sample or genetic information sample) from a subject. Assays for detections polymorphisms or mutations fall into several categories, including, but not limited to direct sequencing assays, fragment polymorphism assays, hybridization assays, and computer based data analysis. Protocols and commercially available kits or services for performing multiple variations of these assays are available. In some embodiments, assays are performed in combination or in hybrid (*e.g.,* different reagents or technologies from several assays are combined to yield one assay).

### A. Direct sequencing Assays

In some embodiments of the present invention, genomic profiles are generated using a direct sequencing technique. In these assays, DNA samples are first isolated from a subject using any suitable method. In some embodiments, the region of interest is cloned into a suitable vector and amplified by growth in a host cell (*e.g.,* a bacteria). In other embodiments, DNA in the region of interest is amplified using PCR.

Following amplification, DNA in the region of interest (*e.g.*, the region containing the SNP or mutation of interest) is sequenced using any suitable method, including but not limited to manual sequencing using radioactive marker nucleotides, or automated sequencing. The results of the sequencing are displayed using any suitable method. The sequence is examined and the presence or absence of a given SNP or mutation is determined.

### B. Fragment Length Polymorphism Assays

In some embodiments of the present invention, genomic profiles are generated using a fragment length polymorphism assay. In a fragment length polymorphism assay, a unique DNA banding pattern based on cleaving the DNA at a series of positions is generated using an enzyme (*e.g.,* a restriction enzyme or a CLEAVASE I [Third Wave Technologies, Madison, WI] enzyme). DNA fragments from a sample containing a SNP or a mutation will have a different banding pattern than wild type.

### 1. RFLP Assay

In some embodiments of the present invention, a genomic profile is generated using a restriction fragment length polymorphism assay (RFLP). The region of interest is first isolated using PCR. The PCR products are then cleaved with restriction enzymes known to give a unique length fragment for a given polymorphism. The restriction-enzyme digested PCR products are separated by agarose gel electrophoresis and visualized by ethidium bromide staining. The length of the fragments is compared to molecular weight markers and fragments generated from wild-type and mutant controls.

### 2. CFLP Assay

In other embodiments, a genomic profile is generated using a CLEAVASE fragment length polymorphism assay (CFLP; Third Wave Technologies, Madison, WI; *See e.g.,* U.S. Patent Nos. 5,843,654; 5,843,669; 5,719,208; and 5,888,780; each of which is herein incorporated by reference). This assay is based on the observation that when single strands of DNA fold on themselves, they assume higher order structures that are highly individual to the precise sequence of the DNA molecule. These secondary structures involve partially duplexed regions of DNA such that single stranded regions are juxtaposed with double stranded DNA hairpins. The CLEAVASE I enzyme, is a structure-specific, thermostable nuclease that recognizes and cleaves the junctions between these single-stranded and double-stranded regions.

The region of interest is first isolated, for example, using PCR. Then, DNA strands are separated by heating. Next, the reactions are cooled to allow intrastrand secondary structure to form. The PCR products are then treated with the CLEAVASE I enzyme to generate a series of fragments that are unique to a given SNP or mutation. The CLEAVASE enzyme treated PCR products are separated and detected (*e.g.,* by agarose gel electrophoresis) and visualized (*e.g.,* by ethidium bromide staining). The length of the fragments is compared to molecular weight markers and fragments generated from wild-type and mutant controls.

### C. Hybridization Assays

In preferred embodiments of the present invention, genomic profiles are generated using a hybridization assay. In a hybridization assay, the presence of absence of a given SNP or mutation is determined based on the ability of the DNA from the sample to hybridize to a complementary DNA molecule (*e.g.*, a oligonucleotide probe). A variety of hybridization assays using a variety of technologies for hybridization and detection are available. A description of a selection of assays is provided below.

### 1. Direct Detection of Hybridization

In some embodiments, hybridization of a probe to the sequence of interest (*e.g.*, a SNP or mutation) is detected directly by visualizing a bound probe (*e.g.,* a Northern or Southern assay; *See e.g.,* Ausabel *et al.* (eds.), Current Protocols in Molecular Biology, John Wiley & Sons, NY [1991]). In a these assays, genomic DNA (Southern) or RNA (Northern) is isolated from a subject. The DNA or RNA is then cleaved with a series of restriction enzymes that cleave infrequently in the genome and not near any of the markers being assayed. The DNA or RNA is then separated (*e.g.,* on an agarose gel) and transferred to a membrane. A labelled (*e.g.,* by incorporating a radionucleotide) probe or probes specific for the SNP or mutation being detected is allowed to contact the membrane under a condition or low, medium, or high stringency conditions. Unbound probe is removed and the presence of binding is detected by visualizing the labelled probe.

### 2. Detection of Hybridization Using "DNA Chip" Assays

In some embodiments of the present invention, genomic profiles are generated using a DNA chip hybridization assay. In this assay, a series of oligonucleotide probes are affixed to a solid support. The oligonucleotide probes are designed to be unique to a given SNP or mutation. The DNA sample of interest is contacted with the DNA "chip" and hybridization is detected.

In some embodiments, the DNA chip assay is a GeneChip (Affymetrix, Santa Clara, CA; *See e.g.,* U.S. Patent Nos. 6,045,996; 5,925,525; and 5,858,659; each of which is herein incorporated by reference) assay. The GeneChip technology uses miniaturized, high-density arrays of oligonucleotide probes affixed to a "chip." Probe arrays are manufactured by Affymetrix's light-directed chemical synthesis process, which combines solid-phase chemical synthesis with photolithographic fabrication techniques employed in the semiconductor industry. Using a series of photolithographic masks to define chip exposure sites, followed by specific chemical synthesis steps, the process constructs high-density arrays of oligonucleotides, with each probe in a predefined position in the array. Multiple probe arrays are synthesized simultaneously on a large glass wafer. The wafers are then diced, and individual probe arrays are packaged in injection-molded plastic cartridges, which protect them from the environment and serve as chambers for hybridization.

The nucleic acid to be analyzed is isolated, amplified by PCR, and labeled with a fluorescent reporter group. The labeled DNA is then incubated with the array using a fluidics station. The array is then inserted into the scanner, where patterns of hybridization are detected. The hybridization data are collected as light emitted from the fluorescent reporter groups already incorporated into the target, which is bound to the probe array. Probes that perfectly match the target generally produce stronger signals than those that have mismatches. Since the sequence and position of each probe on the array are known, by complementarity, the identity of the target nucleic acid applied to the probe array can be determined.

In other embodiments, a DNA microchip containing electronically captured probes (Nanogen, San Diego, CA) is utilized (*See e.g.,* U.S. Patent Nos. 6,017,696; 6,068,818; and 6,051,380; each of which are herein incorporated by reference). Through the use of microelectronics, Nanogen's technology enables the active movement and concentration of charged molecules to and from designated test sites on its semiconductor microchip. DNA capture probes unique to a given SNP or mutation are electronically placed at, or "addressed" to, specific sites on the microchip. Since DNA has a strong negative charge, it can be electronically moved to an area of positive charge.

First, a test site or a row of test sites on the microchip is electronically activated with a positive charge. Next, a solution containing the DNA probes is introduced onto the microchip. The negatively charged probes rapidly move to the positively charged sites, where they concentrate and are chemically bound to a site on the microchip. The microchip is then washed and another solution of distinct DNA probes is added until the array of specifically bound DNA probes is complete.

A test sample is then analyzed for the presence of target DNA molecules by determining which of the DNA capture probes hybridize, with complementary DNA in the test sample (*e.g*., a PCR amplified gene of interest). An electronic charge is also used to move and concentrate target molecules to one or more test sites on the microchip. The electronic concentration of sample DNA at each test site promotes rapid hybridization of sample DNA with complementary capture probes (hybridization may occur in minutes). To remove any unbound or nonspecifically bound DNA from each site, the polarity or charge of the site is reversed to negative, thereby forcing any unbound or nonspecifically bound DNA back into solution away from the capture probes. A laser-based fluorescence scanner is used to detect binding,

In still further embodiments, an array technology based upon the segregation of fluids on a flat surface (chip) by differences in surface tension (ProtoGene, Palo Alto, CA) is utilized (*See e.g.,* U.S. Patent Nos. 6,001,311; 5,985,551; and 5,474,796; each of which is herein incorporated by reference). Protogene's technology is based on the fact that fluids can be segregated on a flat surface by differences in surface tension that have been imparted by chemical coatings. Once so segregated, oligonucleotide probes are synthesized directly on the chip by ink-jet printing of reagents. The array with its reaction sites defined by surface tension is mounted on a X/Y translation stage under a set of four piezoelectric nozzles, one for each of the four standard DNA bases. The translation stage moves along each of the rows of the array and the appropriate reagent is delivered to each of the reaction site. For example, the A amidite is delivered only to the sites where amidite A is to be coupled during that synthesis step and so on. Common reagents and washes are delivered by flooding the entire surface and then removing them by spinning.

DNA probes unique for the SNP or mutation of interest are affixed to the chip using Protogene's technology. The chip is then contacted with the PCR-amplified genes of interest. Following hybridization, unbound DNA is removed and hybridization is detected using any suitable method (*e.g.,* by fluorescence de-quenching of an incorporated fluorescent group).

In yet other embodiments, a "bead array" is used for the generation of genomic profiles (Illumina, San Diego, CA; *See e.g.,* PCT Publications WO 99/67641 and WO 00/39587, each of which is herein incorporated by reference). Illumina uses a BEAD ARRAY technology that combines fiber optic bundles and beads that self-assemble into an array. Each fiber optic bundle contains thousands to millions of individual fibers depending on the diameter of the bundle. The beads are coated with an oligonucleotide specific for the detection of a given SNP or mutation. Batches of beads are combined to form a pool specific to the array. To perform an assay, the BEAD ARRAY is contacted with a prepared subject sample (e.g., DNA). Hybridization is detected using any suitable method.

### 3. Enzymatic Detection of Hybridization

In some embodiments of the present invention, genomic profiles are generated using a assay that detects hybridization by enzymatic cleavage of specific structures (INVADER assay, Third Wave Technologies; *See e.g.,* U.S. Patent Nos. 5,846,717; 6,001,567; 5,985,557; and 5,994,069; each of which is herein incorporated by reference). The INVADER assay detects specific DNA and RNA sequences by using structure-specific enzymes to cleave a complex formed by the hybridization of overlapping oligonucleotide probes. Elevated temperature and an excess of one of the probes enable multiple probes to be cleaved for each target sequence present without temperature cycling. These cleaved probes then direct cleavage of a second labeled probe. The secondary probe oligonucleotide can be 5'-end labeled with fluorescein that is quenched by an internal dye. Upon cleavage, the de-quenched fluorescein labeled product may be detected using a standard fluorescence plate reader.

The INVADER assay detects specific mutations and SNPs in unamplified genomic DNA. The isolated DNA sample is contacted with the first probe specific either for a SNP/mutation or wild type sequence and allowed to hybridize. Then a secondary probe, specific to the first probe, and containing the fluorescein label, is hybridized and the enzyme is added. Binding is detected by using a fluorescent plate reader and comparing the signal of the test sample to known positive and negative controls.

In some embodiments, hybridization of a bound probe is detected using a TaqMan assay (PE Biosystems, Foster City, CA; *See e.g.,* U.S. Patent Nos. 5,962,233 and 5,538,848, each of which is herein incorporated by reference). The assay is performed during a PCR reaction. The TaqMan assay exploits the 5'-3' exonuclease activity of the AMPLITAQ GOLD DNA polymerase. A probe, specific for a given allele or mutation, is included in the PCR reaction. The probe consists of an oligonucleotide with a 5'-reporter dye (e.g., a fluorescent dye) and a 3'-quencher dye. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In still further embodiments, genomic profiles are generated using the SNP-IT primer extension assay (Orchid Biosciences, Princeton, NJ; *See e.g.,* U.S. Patent Nos. 5,952,174 and 5,919,626, each of which is herein incorporated by reference). In this assay, SNPs are identified by using a specially synthesized DNA primer and a DNA polymerase to selectively extend the DNA chain by one base at the suspected SNP location. DNA in the region of interest is amplified and denatured. Polymerase reactions are then performed using miniaturized systems called microfluidics. Detection is accomplished by adding a label to the nucleotide suspected of being at the SNP or mutation location. Incorporation of the label into the DNA can be detected by any suitable method (*e.g.,* if the nucleotide contains a biotin label, detection is via a fluorescently labelled antibody specific for biotin).

### D. Mass Spectroscopy Assay

In some embodiments, a MassARRAY system (Sequenom, San Diego, CA.) is used to generate a genomic profile (*See e.g.,* U.S. Patent Nos. 6,043,031; 5,777,324; and 5,605,798; each of which is herein incorporated by reference). DNA is isolated from blood samples using standard procedures. Next, specific DNA regions containing the mutation or SNP of interest, about 200 base pairs in length, are amplified by PCR. The amplified fragments are then attached by one strand to a solid surface and the non-immobilized strands are removed by standard denaturation and washing. The remaining immobilized single strand then serves as a template for automated enzymatic reactions that produce genotype specific diagnostic products.

Very small quantities of the enzymatic products, typically five to ten nanoliters, are then transferred to a SpectroCHIP array for subsequent automated analysis with the SpectroREADER mass spectrometer. Each spot is preloaded with light absorbing crystals that form a matrix with the dispensed diagnostic product. The MassARRAY system uses MALDI-TOF (Matrix Assisted Laser Desorption Ionization - Time of Flight) mass spectrometry. In a process known as desorption, the matrix is hit with a pulse from a laser beam. Energy from the laser beam is transferred to the matrix and it is vaporized resulting in a small amount of the diagnostic product being expelled into a flight tube. As the diagnostic product is charged when an electrical field pulse is subsequently applied to the tube they are launched down the flight tube towards a detector. The time between application of the electrical field pulse and collision of the diagnostic product with the detector is referred to as the time of flight. This is a very precise measure of the product's molecular weight, as a molecule's mass correlates directly with time of flight with smaller molecules flying faster than larger molecules. The entire assay is completed in less than one thousandth of a second, enabling samples to be analyzed in a total of 3-5 second including repetitive data collection. The SpectroTYPER software then calculates, records, compares and reports the genotypes at the rate of three seconds per sample.

### E. Computer-Based Data Analysis

In some embodiments of the present invention, perioperative genomic profiles are generated using computer-based data analysis of a genetic information sample (*e.g.,* stored nucleic acid sequence information). A sample is collected from a subject at any time (*e.g.*, at birth), sequence information is generated (*e.g.*, through DNA sequencing), and the information is stored (*e.g.,* as digital information on a portable chip). During the perioperative, period, the subject's sequence information is scanned by a computer program for the pre-selected markers. A report (*e.g.,* a perioperative genomic profile) is generated.

### III. Analysis and Delivery of Data

In some preferred embodiments of the present invention, the information generated by perioperative genomic profiling is distributed in an coordinated and automated fashion. A diagram outlining the flow of information in some embodiments of the present invention is shown in Figure 1. Figure 1 shows that certain criteria may be considered in deciding if, and how, to generate a perioperative genomic profile. Specifically, a determination is made whether a subject scheduled for surgery is a candidate for genomic profiling (*e.g.,* will undergo procedures that would be altered depending on the information content of a genomic profile). Analytical validity is also assessed. In particular, the method used to generate the genomic profile is selected based on its ability to provide useful information for a particular application and its practicality (*e.g.*, safety for the operating technician, cost-effectiveness, efficiency). Lastly, the validity of the particular profiling assay is assessed for clinical utility (*e.g.*, the ability to provide a prediction of a phenotype related to the genotype). Once a suitable candidate subject, assay techniques, and assay are selected, a genomic profile is generated by subjecting a genomic specimen (*e.g.,* tissue sample or pre-determined genetic information) from the subject to the assay technique using the particular genetic markers selected. For example, a subject may provide a sample (*e.g.*, blood, tissue, or genetic information) perioperatively (*e.g.,* several weeks prior to surgery in a clinician's office or in the emergency room) and the sample is used to generate a genomic profile using the appropriate assay. In some embodiments of the present invention, the data is generated, processed, and/or managed using electronic communications systems (*e.g.,* Internet-based methods).

In some embodiments, a computer-based analysis program is used to translate the raw data generated by the genomic profile (*e.g.*, the presence or absence of a given SNP or mutation) into data of predictive value for the clinician (*e.g.,* probability of abnormal pharmacological response, presence of underlying disease, or differential diagnosis of known disease). The clinician (*e.g.*, surgeon or anesthesiologist) can access the predictive data using any suitable means. Thus, in some preferred embodiments, the present invention provides the further benefit that the clinician, who is not likely to be trained in genetics or molecular biology, need not understand the raw data of the genomic profile. The data is presented directly to the clinician in its most useful form. The clinician is then able to immediately utilize the information in order to optimize the perioperative care of the subject.

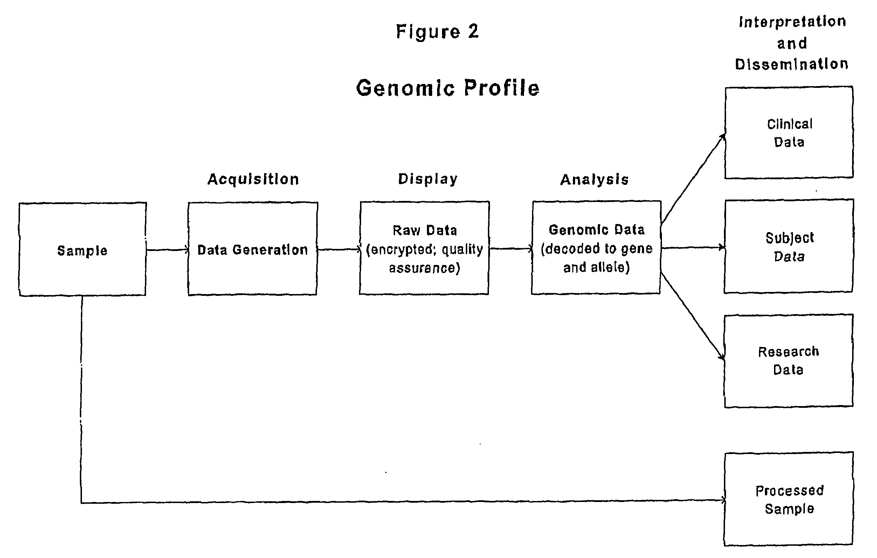

The present invention contemplates any method capable of receiving, processing, and transmitting the information to and from medical personal and subject. Figure 2 illustrates the transformation of a sample (*e.g.,* tissue sample or genetic information) into data useful for the clinician, subject, or researcher. For example, in some embodiments of the present invention, a sample is obtained from a subject and submitted to a genomic profiling service (*e.g.,* clinical lab at a medical facility, genomic profiling business, etc.) to generate raw data. Where the sample comprises a tissue or other biological sample, the subject may visit a medical center to have the sample obtained and sent to the genomic profiling center, or subjects may collect the sample themselves and directly send it to a genomic profiling center. Where the sample comprises previously determined genetic information (*e.g.,* sequence information, SNP or mutation information, etc.), the information may be directly sent to the genomic profiling service by the subject (*e.g.,* a information card containing the genetic information may be scanned by a computer and the data transmitted to a computer of the genomic profiling center using an electronic communication systems). Once received by the genomic profiling service, the sample is processed and a genomic profile is produced (*i.e*., genomic data), specific for the medical or surgical procedure the subject will undergo.

The genomic profile data is then prepared in a format suitable for interpretation by a treating clinician. For example, rather than providing raw sequence data, the prepared format may represent a risk assessment for various treatment options the clinician may use or as recommendations for particular treatment options. The data may be displayed to the clinician by any suitable method. For example, in some embodiments, the genomic profiling service generates a report that can be printed for the clinician (*e.g.*, at the point of care) or displayed to the clinician on a computer monitor.

One exemplary embodiment of such a system finds use for emergency surgery conditions. For example, a sample from a subject may be taken immediately upon first contact of medical personnel with the subject in need of emergency treatment (*e.g.,* taken by an emergency response team at the site of an accident). The sample may be processed using the appropriate detection technique in an emergency response vehicle while the subject is in transport to a medical center emergency room. The data generated by the assay may converted to a genomic profile in a computer system of the emergency vehicle or may be transmitted to distant computer system for processing. Once the genomic profile is generated, a report is sent to the treating physician so the pre-surgical preparation can be conducted (*e.g.,* selection of proper drugs) prior to the arrival of the subject in the emergency room or so that procedures can be changed during surgery if the information arrives after treatment begins.

In some embodiments, the genomic information (*e.g.,* tissue sample or genetic information) is first analyzed at a the point of care or at a regional facility. The raw data is then sent to a central processing facility for further analysis into genomic data and clinician or patient data. The central processing facility provides the advantage of privacy (all genomic data is stored in a central facility with uniform security protocols), speed, and uniformity of data analysis. The central processing facility can then control the fate of the data following surgery. For example, using an electronic communication system, the central facility can provide data to the clinician, the subject, or researchers.

Following the medical or surgical procedure, the subject's sample and the data generated by the genomic profile can follow one of several paths. The fate of the sample and the genomic data is driven by the subject, who is given a menu (*e.g.,* electronically) of choices. The sample may be destroyed, archived, or donated for research use. The genomic data may be destroyed without being seen by anyone other than the clinician (or being seen by the clinician in a limited manner). Such destruction may be desired to maintain the privacy of the subject. In the case of a human subject, the subject may request access to the data for future use. In the case of a non-human subject, the subject' care giver (*e.g.,* owner) may access the data for future use. In some embodiments, the subject may be able to directly access the data using the electronic communication system. The subject may chose further intervention or counseling based on the results. In some embodiments, the data is used for research use. For example, the data may be used to further optimize the inclusion or elimination of markers in the genomic profile.

The present invention provides a unique system for specifically monitoring and tracking empirical results. For example, the success or failure of particular treatment options, selected using the genomic profiles of the present invention, can be compiled in a database to empirically determine more accurate systems for generating and reporting profiles. Such data may indicate that certain markers used in an assay are particularly predictive of an outcome or that other markers, previously considered predictive, have limited value. Using this monitoring and tracking system, the genomic profiles of the present invention continuously evolve to improve results. The use of such systems by medical facilities improves the standard of care, while creating more efficiency and predictability in the management of medical businesses. The present invention thus provides a coordinated, timely, and cost effective system for obtaining, analyzing, and distributing life-saving information.

### EXPERIMENTAL

The following example is provided in order to demonstrate and further illustrate certain preferred embodiments and aspects of the present invention and is not to be construed as limiting the scope thereof.

In the experimental disclosure which follows, the following abbreviations apply: µM (micromolar); mol (moles); mmol (millimoles); µmol (micromoles); nmol (nanomoles); g (grams); mg (milligrams); µg (micrograms); ng (nanograms); 1 or L (liters); ml (milliliters); µl (microliters); cm (centimeters); mm (millimeters); µm (micrometers); nm (nanometers); °C (degrees Centigrade); U (units), mU (milliunits); min. (minutes); % (percent); PEG (poly ethylene glycol); kb (kilobase); bp (base pair); PCR (polymerase chain reaction); Third Wave Technologies (Third Wave Technologies, Madison, WI); Beckman (Beckman Coulter, Fullerton, CA); Gentra Systems (Gentra Systems, Minneapolis, MN); MJ Research (MJ Research, Watertown, MA); and NEB (New England Biolabs, Beverly, MA).

### PERIOPERATIVE GENOMIC SCREENING FOR ANESTHESIA MARKERS

This Example illustrates the generation of a profile for perioperative genomic screening for a patient's response to anesthesia and related medications. Consenting adults, presenting for outpatient surgery, are screened for the variables presented in Tables 1-4. Table 1 lists markers for butyrylcholinesterase deficiency (mutations in the butyrylcholinesterase gene (BChE)). Table 2 lists markers indicative of poor debrisoquine metabolism. Table 3 lists markers indicative of increased risk for thrombus formation. Mutations are in the methylene tetrahyrofolate reductase gene (MTHFR), the methionine synthase gene (MS), the cystathionine β-synthase gene (CBS), the factor 5 Leiden gene (F 5 Leiden), and the prothrombin gene. Table 4 lists markers indicative of increased risk for malignant hyperthermia.

Patients provide a 10 ml blood sample. Leucocyte DNA is extracted from the buffy coat of citrate anticoagulated blood using the Gentra Systems Puregene Isolation kit according to manufacturers instructions. DNA samples are quantitated by UV spectroscopy using a Beckman DU06 spectrophotometer.

The DNA is screened for the mutations and polymorphisms described above using a PCR-restriction fragment length polymorphism (RFLP) assay using standard methods. The DNA in the region of interest is amplified using PCR. PCR reactions are performed on a MJ Research PTC-200 thermocycler. Fragments are next cut with restriction enzymes (NEB) known to give a unique length fragment for a given polymorphism. The restriction-enzyme digested PCR products are separated by agarose gel electrophoresis and visualized by ethidium bromide staining. The length of the fragments is compared to molecular weight markers (NEB)

In addition to the RFLP analysis, DNA samples are analyzed using a flap endonuclease assay (INVADER, Third Wave Technologies; *See e.g.,* Kwiatkowski *et al.,* Molecular Diagnosis, 4:353 [1999]). Separate reactions are performed for mutant and wild-type alleles. Each reaction is performed in triplicate. For each allele, 8 µl of primary reaction mixture (5µl 16% PEG, 2 µl 100 mM MOPS, and 1 µl 0.5 µM primary specific oligonucleotide) is aliquoted into 96 well reaction microplates (MJ Research). Control reactions are also performed, including no DNA target, and wild type, mutant, and heterozygous DNA control samples obtained from known genomic controls amplified by PCR. Samples are incubated for 5 min at 95°C in a thermocycler (MJ Research PTC-200). Then, the temperature is lowered to 63°C and 5µl of the appropriate probe reaction mixture is added to each well. The samples are then incubated at 63°C for 120 min.

Secondary reactions are next performed using common reagents for both wild type and mutant assays. The incubated reactions are cooled to 56°C and 5µl of the secondary reaction mixture is added (1 µl H₂O, 0.5µl 100 mM MOPS, 0.5µl 75 mM MgCl₂, 1µl 30 µl arrestor, 1 µl secondary DNA target, and 1µl FRET probe). The reactions are incubated at 56°C for 120 min. The reactions are stopped by the addition of 175 µl 10 mM EDTA and 180 µl of each reaction is transferred to a microtiter plate to be read in a CytoFluor Series 4000 fluorescent multiwell plate reader with an excitation wavelength of 485 nM and an emission wavelength of 530 nM.

Disparities between the RFLP and flap endonulease assays are resolved by direct sequencing using a ABI model 377 automated sequencer using appropriate fluorescent dye-terminators.

Results of the genomic profile are used to make appropriate decisions about patient care, including choice of analgesics and anesthetics, post surgical monitoring, and additional medications or treatments.

**Table 1**

| **Butyrylcholinesterase Deficiency Markers** | | | |
|---|---|---|---|
| Gene | Mutation | % Incidence (homozygote/heterozygote) | Reference |
| BChE | A209G | .05/4 "atypical" | Cell. Mol. Neurobiol., 11:79 [1991] |
| BChE | G1615A | 1.3/22 "K-Variant" | Cell. Mol. Neurobiol., 11:79 [1991] |

**Table 2**

| **Poor Debrisioquine Metabolism Markers** | | | |
|---|---|---|---|
| Gene | Mutation | % Incidence (homozygote/heterozygote) | Reference |
| CYP2D6 | G1934A | 66% of poor metabolizers | Am. J. Hum. Genet., 60:284 [1997] |
| CYP2D6 | deletion | 17% of poor metabolizers | Am. J. Hum. Genet., 60:284 [1997] |
| CYP26D | A2637del | 4% of poor metabolizers | Am. J. Hum. Genet., 60:284 [1997] |
| CYP2D6 | T1975del | % of poor metabolizers | Am. J. Hum. Genet., 60:284 [1997] |

**Table 3**

| **Markers for Thrombus Formation** | | | |
|---|---|---|---|
| Gene | Mutation | % Incidence (homozygote/heterozygote) | Reference |
| MTHFR | 677T | 12%/>30% | Nature Genet., 10:111 [1995] |
| MTHFR | A1298C | | Nature Genet., 10:111 [1995] |
| MS | A2756 | 2%/35% | Genet. Epidemiol., 17:298 [1999] |
| CBS | Intron 7 68 bp insertion | 1%/12% | Am. J. Hum. Genet., 59:1262 [1996] |
| F 5 Leiden | G1691A | 6% of population | New Eng. J. Med., 336:399 [1997] |
| Prothrombin | G20210A | 2% of population | New Eng. J. Med., 341:801 [1999] |

**Table 4**

| **Markers for Malignant Hyperthermia** | | | |
|---|---|---|---|
| Gene | Mutation | % Incidence (homozygote/heterozygote) | Reference |
| RYR1 | G6502A | 7% of MH cases | Hum. Mol. Genet., 8:2055 [1999] |
| RYR1 | G1021A | 6-10% of MH cases | Hum. Mol. Genet., 8:2055 [1999] |
| RYR1 | C1840T | 4% of MH cases | Hum. Mol. Genet., 8:2055 [1999] |
| RYR1 | C6487T | 4% of MH cases | Hum. Mol. Genet., 8:2055 [1999] |
| RYR1 | G7303A | 4% of MH cases | Hum. Mol. Genet., 8:2055 [1999] |
| RYR1 | C7373A | 4% of MH cases | Hum. Mol. Genet., 8:2055 [1999] |
| CACNA1S | G3257A | 4 families | Am. J. Hum. Genet., 60:1316 [1997] |
| CPT2 | C2023T | 3 families | Am. J. Hum. Genet., 20 A5 [1998] |

## Claims

1. A method comprising:
a) providing:
i) a sample obtained from a perioperative subject; and
ii) an assay for detecting two or more genetic markers, wherein said markers comprise a mutation in two or more genes selected from the group consisting of BChE, CYP2D6, MTHFR, MS, CBS, F 5 Leiden, Prothrombin, RYR1, CACNA1S, and CPT 2; and
b) subjecting said sample to said assay to generate a genomic profile for use in selection of conditions for anesthesia during a surgical procedure.

2. The method of Claim 1, wherein said anesthesia is a general anesthesia.

3. The method of Claim 1, wherein said anesthesia is a regional anesthesia.

4. The method of Claim 1, wherein said surgical procedure is non-invasive surgery.

5. The method of Claim 1, wherein said surgical procedure is invasive surgery.

6. The method of Claim 1, wherein said genomic profile comprises information pertaining to a pharmacodynamic risk.

7. The method of Claim 1, wherein said genomic profile comprises information pertaining to a pharmacokinetic risk.

8. The method of Claim 1, wherein said genomic profile comprises a presymptomatic diagnosis.

9. The method of Claim 1, wherein said genomic profile comprises information pertaining to differential diagnosis of co-existing diseases.

10. The method of Claim 9, wherein said pharmacological response is to an anesthetic.

11. The method of Claim 9, wherein said conditions for said procedure is the choice of anesthetic.

## Patentansprüche

1. Verfahren das aufweist:
a) Bereitstellung
i) einer von einem perioperativen Subjekt erhaltene Probe; und
ii) eines Assays zur Feststellung von zwei oder mehr genetischen Markern, wobei die Marker eine Mutation in zwei oder mehr Genen ausgewählt aus der Gruppe bestehend aus BChE, CYP2D6, MTHFR, MS, CBS, F 5 Leiden, Prothrombin, RYR1, CACNA1S und CPT 2 aufweisen; und
b) Einbringen der Probe in den Assay zur Erzeugung eines genomischen Profils zur Verwendung in der Auswahl von Narkosebedingungen bei einer chirurgischen Behandlung.

2. Verfahren nach Anspruch 1, **dadurch gekennzeichnet, dass** die Narkose eine Vollnarkose ist.

3. Verfahren nach Anspruch 1, **dadurch gekennzeichnet, dass** die Narkose eine Teilnarkose ist.

4. Verfahren nach Anspruch 1, **dadurch gekennzeichnet, dass** die chirurgische Behandlung eine nicht-invasive chirurgische Behandlung ist.

5. Verfahren nach Anspruch 1, **dadurch gekennzeichnet, dass** die chirurgische Behandlung eine invasive chirurgische Behandlung ist.

6. Verfahren nach Anspruch 1, **dadurch gekennzeichnet, dass** das genomische Profil Informationen in Bezug auf ein pharmakodynamisches Risiko aufweist.

7. Verfahren nach Anspruch 1, **dadurch gekennzeichnet, dass** das genomische Profil Informationen in Bezug auf ein pharmakokinetisches Risiko aufweist.

8. Verfahren nach Anspruch 1, **dadurch gekennzeichnet, dass** das genomische Profil eine vorsymptomatische Diagnose aufweist.

9. Verfahren nach Anspruch 1, **dadurch gekennzeichnet, dass** das genomische Profil Informationen in Bezug auf verschiedene Diagnosen von koexistierenden Krankheiten aufweist.

10. Verfahren nach Anspruch 9, **dadurch gekennzeichnet, dass** sich die pharmakologische Reaktion auf ein Narkosemittel bezieht.

11. Verfahren nach Anspruch 9, **dadurch gekennzeichnet, dass** die Bedingungen für die genannte Verfahrensweise die Auswahl des Narkosemittels ist.

## Revendications

1. Procédé comprenant les étapes consistant à :
a) fournir :
i) un échantillon obtenu à partir d'un sujet périopératoire ; et
ii) un essai pour détecter deux marqueurs génétiques ou plus, dans lequel lesdits marqueurs comprennent une mutation dans deux gènes ou plus choisis dans le groupe constitué par BChE, CYP2D6, MTHFR, MS, CBS, F5 Leiden, la prothrombine, RYR1, CACNA1S, et CPT2 ; et
b) soumettre ledit échantillon audit essai pour générer un profil génomique pour utilisation dans la sélection de conditions pour l'anesthésie durant une procédure chirurgicale.

2. Procédé selon la revendication 1, dans lequel ladite anesthésie est une anesthésie générale.

3. Procédé selon la revendication 1, dans lequel ladite anesthésie est une anesthésie locale.

4. Procédé selon la revendication 1, dans lequel ladite procédure chirurgicale est une chirurgie non invasive.

5. Procédé selon la revendication 1, dans lequel ladite procédure chirurgicale est une chirurgie invasive.

6. Procédé selon la revendication 1, dans lequel ledit profil génomique comprend des informations relatives à un risque pharmacodynamique.

7. Procédé selon la revendication 1, dans lequel ledit profil génomique comprend des informations relatives à un risque pharmacocinétique.

8. Procédé selon la revendication 1, dans lequel ledit profil génomique comprend un diagnostic présymptomatique.

9. Procédé selon la revendication 1, dans lequel ledit profil génomique comprend des informations relatives à un diagnostic différentiel de maladies coexistantes.

10. Procédé selon la revendication 9, dans lequel ladite réponse pharmacologique est un anesthésique.

11. Procédé selon la revendication 9, dans lequel lesdites conditions pour ladite procédure est le choix d'anesthésique.
